(19) Europäisches Patentamt
European Patent Office
Office européen des brevets

(11) Publication number: 0 146 924 B1

(12) # EUROPEAN PATENT SPECIFICATION

(45) Date of publication of patent specification: 07.06.89

(51) Int. Cl.$^4$: C 07 D 213/61

(21) Application number: 84115856.1

(22) Date of filing: 19.12.84

(54) **Preparation of difluorpyridine compounds.**

(30) Priority: 23.12.83 US 564800
29.10.84 US 665588

(43) Date of publication of application:
03.07.85 Bulletin 85/27

(45) Publication of the grant of the patent:
07.06.89 Bulletin 89/23

(84) Designated Contracting States:
BE CH DE FR GB IT LI NL SE

(56) References cited:
EP-A-0 063 872
EP-A-0 097 460
EP-A-0 104 715
EP-A-0 120 575
GB-A-1 039 987
US-A-4 031 100

**The file contains technical information submitted after the application was filed and not included in this specification**

(73) Proprietor: THE DOW CHEMICAL COMPANY
2030 Dow Center Abbott Road P.O. Box 1967
Midland Michigan 48640-1967 (US)

(72) Inventor: Little, John C.
5450 Kirkwood Drive, C-2
Concord California 94521 (US)
Inventor: Wilson, Charles A.
Marina Boulevard P.O. Box 1355
Pittsburg California 94565 (US)

(74) Representative: Huber, Bernhard, Dipl.-Chem. et al
Patentanwälte H. Weickmann, Dr. K. Fincke F.A. Weickmann, B. Huber Dr. H. Liska, Dr. J. Prechtel
Möhlstrasse 22 Postfach 860 820
D-8000 München 86 (DE)

EP 0 146 924 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to the preparation of alpha, beta and beta, gamma-difluoropyridine compounds employing potassium fluoride (KF) and/or cesium fluoride (CsF) as the fluorinating agent.

Alkali metal fluorides are well-known agents for the conversion of ring-chlorinated pyridines to the corresponding fluoropyridines. Thus, Finger, *et al.* (J. Org. Chem. *28*, 1666 (1963)), found that KF in dimethyl sulfone at 200°C over a period of time converted 2-chloropyridine to 2-fluoropyridine. Similarly, 2,3,5-trichloro- and 2,3,5,6-tetrachloropyridine gave the 2-fluoro- and 2,6-difluoro-3,5-dichloropyridines.

It is equally well-known that the exchange of chlorine on pyridine for fluorine using the nucleophilic action of fluoride ion very strongly favors replacement at the alpha or gamma positions of chloropyridines, with a beta-chlorine remaining essentially inert. Thus, in addition to the above cases, it has been noted by Chambers, *et al*, (Proc. Chem. Soc. *1964*, 83) that pentachloropyridine, for example, strongly favors exchange at the alpha and gamma-positions when heated to *ca.* 200°C in a polar, aprotic diluent, and only under extreme conditions (anhydrous KF, 400—500°C, 24 hr) does the exchange of the beta (3- and 5-) chlorines occur. Moreover, whenever this exchange at the beta (3- and/or 5-) position has been observed, it has been limited to fully-substituted chloropyridines (cf. GB—A—1 039 987): the above-mentioned 2,3,5,6-tetrachloropyridine (having a hydrogen at the 4-position) gives only decomposition products under these conditions (Chambers, *loc cit.*). In closely-related substitution reactions, a beta-chloropyridine has been found to be 10,000—100,000 times less reactive than the alpha-chloro- or gamma-chloropyridine, and theoretical explanations have been offered (Newkome and Paudler, "Contemporary Heterocyclic Chemistry", New York, John Wiley (1982), pp 262—3).

EP—A—0 063 872 teaches that it is known to react chloropyridines with KF, in the presence or absence of a polar aprotic diluent, in order to replace chlorine by fluorine. This reference discloses on page 5 that when 2,3-dichloro-5-(trichloromethyl)pyridine is allowed to react with KF the chlorine in the 3-position (beta-position) remains unchanged while all the other chlorine atoms are replaced by fluorine. The resulting product is 3-chloro-2-fluoro-5-(trifluoromethyl)pyridine (I). Only in case of 3,4,5-trichloro-2-trichloromethyl-pyridine, a replacement of all Cl atoms takes place due to the activating trichloromethyl-group in ortho-position to Cl.

Similarly, the use of CsF as a fluorinating agent is taught in, e.g., European Patent Applications 104,715 and 97,460. These applications teach what are believed to be the first examples of direct substitution (with fluoride ion) of fluorine for the chlorine on a 3-chloropyridine having hydrogen on the ring. EP 97,460 cites the reaction of CsF with 3-chloro-2-cyano-5-(trifluoromethyl)pyridine, II, to yield the beta-fluoropyridine, III.

II

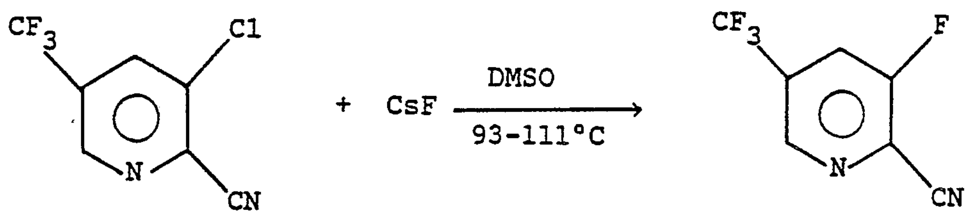


III

In this example, the well-known influence of an adjacent cyano group on an aromatic ring, which powerfully activates a halogen (chlorine) towards substitution (by fluoride), is believed to be operating. Under similar reaction conditions and without the cyano group in O-position replacement of chlorine by fluorine takes place only after a long reaction period and in poor yield.

EP 104,715 discloses that fluoride ion from cesium fluoride in an aprotic diluent will react with 3-chloro-2-fluoro-5-(trifluoromethyl)pyridine (I) to give 2,3-difluoro-5-(trifluoromethyl)pyridine, IV:

I

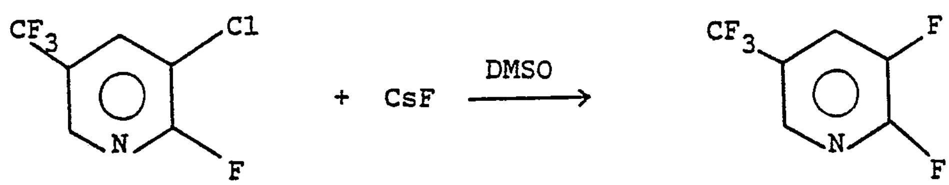


IV

This reference teaches in a preferred embodiment the use of about 50 percent molar excess of CsF in dimethyl sulfoxide (DMSO) diluent at 120—125° for about 48 hours, and the method gives yields of 48—58 percent IV.

The present invention represents a substantial improvement over the process taught in the above EP application 104,715, in that yields are much improved (up to 90 percent or more), reaction times are shorter, and the reaction may be effectively carried out with both CsF and the much less expensive KF. In addition, the new procedure has been shown to be applicable for the preparation of a number of other valuable alpha, beta, and beta, gamma-difluoropyridines from a wide variety of starting materials. It has also been found that the required alpha- or gamma-fluoro-beta-halopyridine starting material may be generated *in situ* from often more readily-available materials.

Subject of the present invention is a method for preparing beta-fluoropyridine compounds according to claim 1.

Preferred embodiments of this method are subject matter of claims 2 to 11.

In accordance with the present invention, alpha, beta (VI) or beta, gamma-difluoropyridines (VIII), useful as herbicide intermediates, can be prepared by causing compounds of the general formula V or VII to react with an effective amount of KF or CsF under substantially anhydrous conditions in a polar aprotic diluent at elevated temperatures while removing the product difluoropyridine VI or VIII, essentially as it is formed. The starting material, V or VII, is optionally added advantageously at essentially the rate of removal of the product difluoropyridine VI or VIII.

V

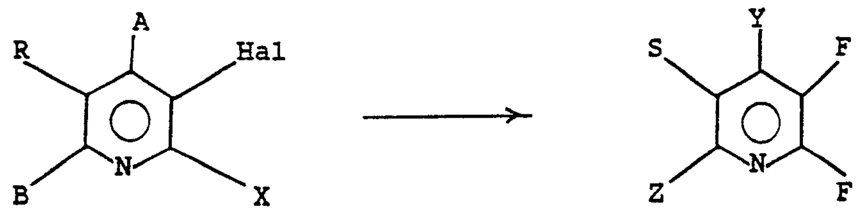

VI

VII

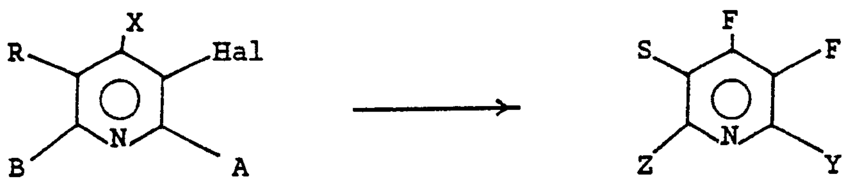

VIII

wherein Hal = Cl, Br, or I; X = F or Hal; A and B are individually H, $CH_3$, $CF_3$, F or Hal; R = H, $CH_3$, $CF_3$, F, Hal or CN; Y is A or F with the proviso that Y becomes F if A is Hal; Z is B or F with the proviso that Z becomes F if B is Hal; and S is R or F with the proviso that S becomes F when R is Hal and either A or B is F or Hal.

In accordance with the present invention, alpha, beta- (X) or beta, gamma-difluoropyridines (XII) are prepared by a process which comprises causing an alpha (IX) or gamma-fluoro-beta-halopyridine (XI), where "halo" represents chloro, bromo or iodo, to react with potassium fluoride or cesium fluoride under substantially anhydrous conditions in a suitable polar aprotic diluent at elevated temperatures, while removing the product difluoropyridine (X or XII) by distillation essentially as it is formed:

IX

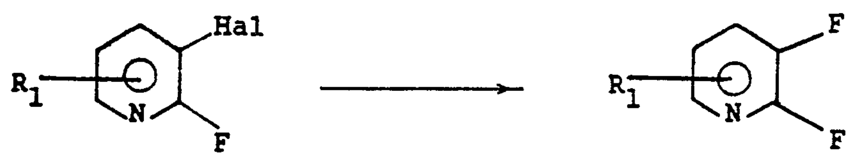

X

or XI

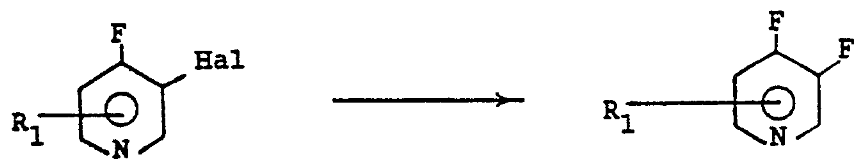

XII

In the above formula (IX—XII), the other substituents (represented by $R_1$) on the ring may be any group or groups which is or are stable under the reaction conditions, such as H, $CH_3$, $CF_3$, F, Cl, Br, I, etc., with the special provision that when the substituent is Cl, Br or I in the alpha- or gamma-positions, it is also converted to fluoro. Preferred reactants have the formula

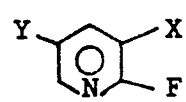

where Y is $CF_3$, Br or Cl and X is Cl or Br.

Of particular interest in the practice of the present invention is a method of preparing 2,3-difluoro-5-(trifluoromethyl)pyridine (IV) from 3-chloro-2-fluoro-5-(trifluoromethyl)pyridine (I):

I

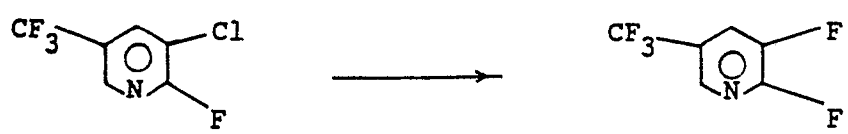

IV

Compound IV is useful as a chemical intermediate in the preparation of 2-(4-((3-fluoro-5-(trifluoromethyl)pyridinyl)-2-oxy)phenoxy propionic acid and agriculturally acceptable derivatives thereof, i.e., salts, esters and amides, which are known herbicidal agents as described in EP 97,460.

In a variant of this invention, the alpha- or gamma-fluoro-beta-halopyridine starting material (IX or XI) may optionally be generated *in situ* by starting with an alpha, beta- (XIII) or beta, gamma-dihalopyridine (XIV) (where "halo" again represents chloro, bromo, or iodo), and taking advantage of the previously-mentioned, well-known tendency of alpha or gamma-halopyridines to exchange with fluoride ion. Under the reaction conditions, use of additional fluoride (KF or CsF) allows the generation of the required alpha- or gamma-fluoro-beta-halopyridine, which is then converted to the desired product alpha, beta- or beta, gamma-difluoropyridine:

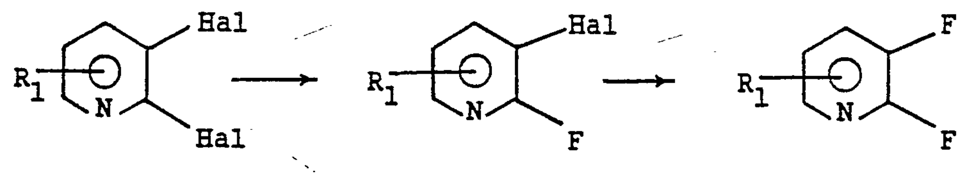

XIII IX X

or

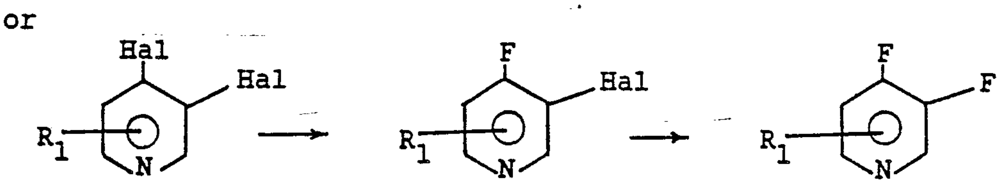

XIV XI XII

Hal = Cl, Br or I

Of particular interest in this variant form are the conversions of 2,3,5-trichloropyridine (XV) and 2,3,5-tribromopyridine (XVIII) to 2,3-difluoro-5-chloropyridine (XVII) and 2,3-difluoro-5-bromopyridine (XX), respectively. The latter two compounds are useful as chemical intermediates in the preparation of 2-(4-((5-chloro-(or bromo)-3-fluoro)pyridinyl-2-oxy)phenoxy propionic acid and agriculturally acceptable derivatives thereof, i.e., for example, salts, esters and amides, which are known herbicidal agents as taught in EPO patent application 83,556:

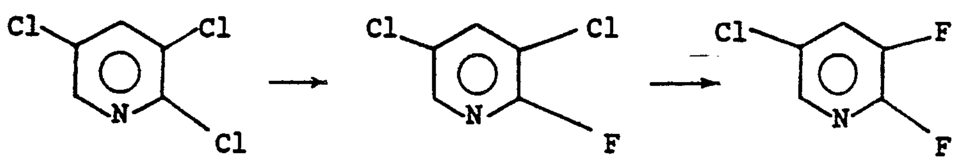

XV XVI XVII

and

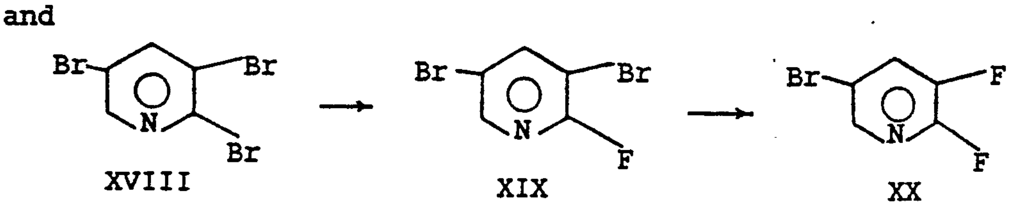

XVIII XIX XX

As mentioned above, if there is a halo (Cl, Br or I) present in the molecule at an alpha or gamma-position, it is likewise converted to fluoro in the final product, assuming that sufficient KF or CsF is present. Thus, 2,3,6-trichloropyridine XXI is transformed into 2,3,6-trifluoropyridine, XXIII.

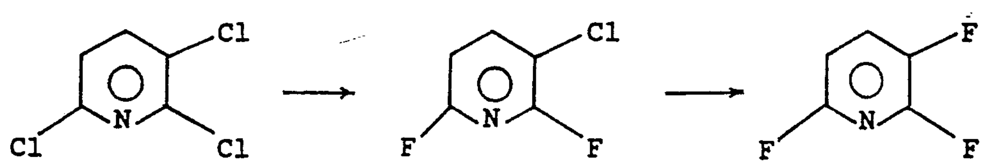

XXI XXII XXIII

Note also that two essentially equivalent "betapositions" may exist in a pyridine molecule, and if the above requirements are met for both "beta-positions" then both are eventually converted to the difluoropyridine derivatives by providing sufficient fluoride salt under suitable reaction conditions. Thus, 2,3,5,6-tetrachloropyridine (XXIV) may be converted stepwise to the corresponding tetrafluoropyridine (XXV) in the presence of at least 4 moles of KF or CsF:

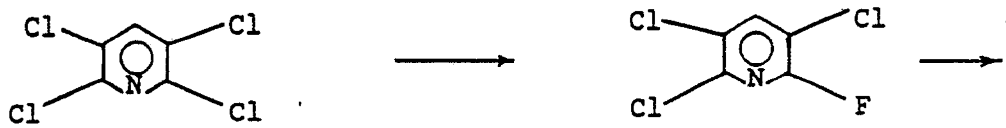

XXIV

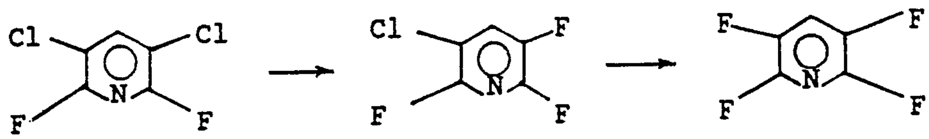

XXV

In the process of this invention, the starting material, IX or XI, is often advantageously added at essentially the rate of removal of the product difluoropyridine (X or XII), thus helping to minimize contact of both the starting material and the product with the reaction medium, in which they tend to decompose.

KF and CsF are the fluorinating agents employed in the present reaction and are commercially available compounds. Substantially anhydrous and finely-divided KF or CsF are preferred. Amorphous or spray-dried forms are particularly preferred. Substantially anhydrous KF and CsF can be prepared, for example, by drying *in vacuo* at 140—250°C for several hours.

Polar aprotic diluents are employed as the reaction medium in the present process. Suitable polar aprotic diluents include dimethylsulfoxide (DMSO), dimethylformamide (DMF), dimethylacetamide, diethylacetamide, methyl isobutyl ketone, hexamethylphosphoric acid triamide, tetramethylurea, sulfolane (tetramethylenesulfone), and N-methylpyrrolidinone (NMP). Preferred diluents include NMP, DMSO and sulfolane.

Optionally, the reaction may be conducted in the presence of

(a) an acid scavenger, such as, an alkali metal carbonate, and/or in the case of employing KF as the fluorinating agent,

(b) a phase-transfer catalyst.

The present reaction is conducted under substantially anhydrous conditions at elevated temperatures of from 50°C up to the boiling point of the solvent. Preferred temperature ranges are from 100°C to 200°C when CsF is used, and from 150°C up to the boiling point of the solvent when KF is used.

Pressures of from 10 mm Hg (1,33 kPa) to 10 atm (1013,2 kPa) may also be employed, with preferred pressures of 50 mm Hg (6,65 kPa) to 1 atm (101,32 kPa).

A fractional distillation system having 1 to 100 theoretical plates is conveniently employed to separate the product from the starting material. A preferred system has 5 to 20 theoretical plates.

The optimum combination of temperature and pressure is actually a function of the particular system being studied and can be determined by routine experimentation. In general the pressure is chosen so as to provide convenient separation of the desired product from the starting material through the fractional distillation system while allowing a reaction temperature (distillation pot temperature) high enough to maintain a satisfactory reaction rate. Experimental determination of the reaction rate can be conveniently judged by observing the drop in the observed reflux temperature in the distillation column from that of the starting material to that of the product.

When 2,3-difluoro-5-(trifluoromethyl)pyridine (IV) is being prepared from 3-chloro-2-fluoro-5-(trifluoromethyl)pyridine (I) using KF and NMP, for example, a pressure of 1 atmosphere (101,32 kPa) and reaction temperatures of 190—205°C are optimally employed. When CsF and DMSO are used to effect the same conversion, a pressure of 150 mm Hg (19,95 kPa) and reactor temperatures of 120—140°C are conveniently used.

Substantially anhydrous reaction conditions are preferred; these may be achieved employing standard drying techniques. For example a typical glass laboratory reactor can be dried by distilling the polar aprotic solvent under a vacuum before addition of the reactants. Optionally, a small amount (5—10 percent by weight of the polar aprotic solvent) of a non-polar solvent such as an aromatic hydrocarbon (toluene, xylene, etc.) may be added to the polar aprotic solvent to aid in the removal of water by azeotropic distillation. Residual water in the reactor system is also often removed by azeotropic distillation with the product X or XII or the starting material IX or XI.

The amount of polar aprotic solvent is not critical but it is advantageous to employ enough solvent to keep the starting material IX or XI in solution at reaction temperatures, generally 2 to 25 parts by weight solvent per part by weight pyridine starting material. The relative proportions of reactants to be employed are not critical because some of the product will be formed when employing any proportion of reactants. The reaction consumes the reactants, however, in the ratio of one mole of fluorinating agent per mole of exchangeable halogen atoms present in the starting material. For example, if 2,3-dichloro-5-(trifluoromethyl)pyridine is the starting material, then about 2 molar equivalents of KF or CsF per molar equivalent pyridine starting material can be employed, and if 3-chloro-2-fluoro-5-(trifluoromethyl)pyridine is the starting material, then about equimolar amounts of KF or CsF and pyridine starting material can be employed. Usually from 0.75 to 1.5 moles of KF or CsF are employed per mole of exchangeable halogen in the pyridine starting material.

In carrying out the present reaction, neither the rate nor the order of addition of the reactants is critical. Usually, the solvent and fluorinating agent are added to an appropriate reaction vessel and the reaction is dried by distilling a small portion of the solvent. The starting material (IX or XI) or precursor compound (XIII or XIV) is then added to the reaction vessel which is thereafter followed by heating of the reaction mixture at a suitable pressure, usually 50 mm Hg to 1 atm (6,65 to 101,32 kPa), to provide convenient separation of the desired product as it is formed. In an especially preferred mode, the starting material is added to the fluorinating agent (KF or CsF) in the solvent under the optimized reaction conditions at about the same molar rate as the formation and removal of the product. If an acid scavenger, a non-polar solvent, or catalyst is employed in the reaction, then they are advantageously added to the solvent/fluorinating agent mixture prior to drying the reactor vessel.

The present reaction is typically conducted in the presence of agitation sufficient to maintain an essentially uniform dispersion of the reactants in the solvent.

Usually the reaction using KF without a catalyst is complete in 16 to 24 hours. Catalysts are optionally employed, when KF is used, to increase the reaction rate. When a catalyst is used with KF, 8 to 16 hours are usually required. When CsF is used, 2 to 8 hours are normally sufficient. Suitable catalysts include phase-transfer catalysts. The catalyst is added to the present reaction mixture in an amount of from 0.0001 to 0.1 mole per mole of pyridine starting material, advantageously from 0.001 to 0.075 molar equivalents and preferably from 0.01 to 0.05 molar equivalents.

Phase-transfer catalysts are well known compounds and include (a) quaternary ammonium or phosphonium salts containing 10 or more carbon atoms and (b) macrocyclic polyethers commonly known as crown ethers. Suitable crown ether catalysts include 18-crown-6; dicyclohexano-18-crown-6; dibenzo-18-crown-6; 15-crown-5. A related species, tris(3,6-dioxaheptyl)amine is also efficaceous. Suitable quaternary ammonium and phosphonium salts include tetran-alkylammonium salts and tetra-n-alkylphosphonium salts. Particular catalysts include benzyltriethylammonium chloride, methyl trioctylammonium chloride, tetra-n-butylammonium chloride, tetra-n-butylammonium hydrogen sulfate, tetranbutylphosphonium chloride and cetyl trimethylammonium bromide. The anion of the phosphonium and ammonium salts of $F^{\ominus}$ or any anion which readily converts to $F^{\ominus}$, such as for example, $Cl^{\ominus}$, $Br^{\ominus}$, $I^{\ominus}$, $OH^{\ominus}$, $OAc^{\ominus}$, etc. Preferred catalysts include 18-crown-6 and cetyltrimethylammonium bromide.

Acid scavengers are optionally employed in the present reaction to consume or inactivate traces of HCl or HF which may be present or generated during the reaction. Suitable acid scavengers include alkali metal carbonates such as anhydrous $K_2CO_3$ and anhydrous $NaCO_3$. A preferred acid scavenger is anhydrous $K_2CO_3$. The acid scavengers are added to the present reaction mixture in an amount of from 0.001 to 0.1 mole per mole of pyridine starting material. Preferably, from 0.03 to 0.05 molar equivalents are employed.

The solvent used in the process of this invention may be distilled to recover it free from impurities and re-used. Alternatively, it has been found that, under certain conditions, the solvent may be re-used without distillation by simply filtering or decanting from the spent potassium or cesium salts and charging fresh KF or CsF. Solvents such as NMP have been re-used as many as 4 times in this manner before further purification was necessary, and additional recycles may be possible.

The following examples illustrate the practice of the present invention and should not be construed as limiting. No attempt has been made to balance any chemical equations described herein. All temperatures are in °C and boiling points are at atmospheric pressure unless otherwise stated.

## Example 1

A one-liter 3-necked flask was equipped with an efficient stirrer, a thermometer, temperature controller, 250 watt infrared heat lamp, a reduced pressure control device and a 2.54 cm OD 7-tray glass Oldershaw (sieve plate) distillation column having a vapor fraction cutter, condenser and a water-jacketed receiver. The flask was charged 550 ml of sulfolane, 43.5 g (0.75 mole) of KF (dried *in vacuo* 48 hours at 140°—160°C and then pulverized) and 5 g of $K_2CO_3$. About 10 ml of sulfolane and water was distilled (b.p. 53°—210°C/13,33 kPa) to dry the system and then the vacuum was released to add 100 g (0.5 mole) of 3-chloro-2-fluoro-5-(trifluoromethyl)pyridine. Heating was resumed at 201°—202°C with vigorous stirring, and the distillation head temperature was observed to drop from 139°C to about 117°C/101,32 kPa over 2 hours, at which point slow distillation (20—40:1 reflux) was begun. Over the next 25 hours, 36 g of liquid, b.p. 109°—122°C/101,32 kPa, containing (by glpc) 30.9 g of desired product and 5.4 g of starting material, was obtained, while the pot temperature was slowly increased to 225°C. The pot was then cooled and the pressure reduced to 250 mm at this point, and an additional 20.6 g of material, containing 18.7 g of desired product and 0.3 g of starting material, was recovered, b.p. 53°—90°C/33,25 kPa. The yield of 2,3-difluoro-5-(trifluoromethyl)pyridine was 60 percent at 94 percent conversion of starting material. Redistillation of this product at atmospheric pressure gave excellent recovery of both desired product and starting material.

Example 2

To the same apparatus as described in Example 1 was charged 515 ml of sulfolane, 43.5 g (0.75 mole) of KF which had been dried at 140°C *in vacuo* for 48 hours and then pulverized, 5 g of $K_2CO_3$ and 5 g of 18-crown-6 ether. The system was dried by distillation of about 25 ml of solvent (b.p. 160°—121°C/73,33 kPa) and then 100 g (0.5 mole) of 2,3-chloro-5-(trifluoromethyl)pyridine was added after releasing the vacuum on the system. The mixture was stirred vigorously at 195°—200°C/101,32 kPa for 1 hour during which time the observed head temperature dropped rapidly to about 114°C, and then distillation was begun. A total of 45.4 g of distillate was taken off over 5 hours at 111°—118°C/101.32 kPa, and glpc analysis indicated the presence of 40.9 g of desired product.

The reaction mixture was then allowed to stir an additional 15 hours at 220°C/101,32 kPa. Distillation yielded an additional 24.4 g of material having a b.p. of 53°—180°C/13,33 kPa and containing 22.9 g of desired product and 0.4 g of starting material as indicated by glpc analysis. The total yield of 2,3-difluoro-5-(trifluoromethyl)pyridine was 71 percent at 98 percent conversion of starting material.

Example 3

To a 2 L flask, equipped as described in Example 1, was charged one liter (1 L) of N-methyl pyrrolidinone (NMP) which was heated under 20 mm at 120°C to dry the system. About 20 ml of NMP and water were removed. The vacuum was released and 100 g (1.7 moles) of KF (dried *in vacuo* 24 hours at 140°—160°C and then pulverized), 20 g of $K_2CO_3$ (anhydrous) and 400 g (2.0 moles) of 3-chloro-2-fluoro-5-(trifluoromethyl)pyridine were added. Heating was resumed with vigorous stirring under a nitrogen atmosphere fed into the top of the reaction flask. The temperature was increased to 190°—195°C and the distillation head temperature was observed to drop from 138°C to 104°C over 2 hours, at which point slow product takeoff (120/1 reflux to takeoff) was begun. Over the next 21 hours 211 g of distillate were recovered. The distillate contained 90 percent 2,3-difluoro-5-(trifluoromethyl)pyridine equivalent to 190.2 g (61 percent yield) based on KF as indicated by glpc.

Example 4

To 3 L flask, equipped as described in Example 1, was charged 1.3 L of N-methyl pyrrolidinone (NMP) which was heated under 20 mmHg (2,66 kPa) at 120°C to dry the system. About 20 ml of NMP and water were removed. The vacuum was released and 116 g (2 moles) of KF (dried *in vacuo* 24 hours and then pulverized), 20 g of $K_2CO_3$ (anhydrous) and 500 g (2.5 moles) of 3-chloro-2-fluoro-5-(trifluoromethyl)-pyridine were added. Heating was resumed with vigorous stirring under a nitrogen atmosphere fed into the top of the reaction flask. The temperature was increased to 190°—195°C and the distillation head temperature was observed to drop from 138°C to 104°C over 2 hours, at which point slow product takeoff (120/1 reflux to takeoff) was begun. Over the next 16 hours, 389 g of distillate were recovered. The distillate contained 75.5 percent 2,3-difluoro-5-(trifluoromethyl)pyridine equivalent to 294 g (80 percent yield) based on KF as indicated by glpc.

Example 5

To a 3 L flask, equipped as described in Example 1, was charged 2 L of N-methyl pyrrolidinone (NMP) which was heated under 20 mm (2,66 kPa) at 120°C to dry the system. About 20 ml of NMP and water were removed. The vacuum was released and 116 g (2 moles) of KF (dried *in vacuo* 24 hours at 140°—160°C and then pulverized), 10 g of $K_2CO_3$ (anhydrous) and 400 g (2.0 moles) of 3-chloro-2-fluoro-5-trifluoromethylpyridine were added to the flask. Heating was resumed with vigorous stirring under a nitrogen atmosphere fed into the top of the reaction flask. The temperature was increased to 190°—195°C and the distillation head temperature was observed to drop from 138°C to 104°C over 2 hours, at which point a slow product takeoff (120/1 reflux to takeoff) was begun. Over the next 20 hours 283 g of distillate were recovered. The distillate contained 88 percent 2,3-difluoro-5-(trifluoromethyl)pyridine equivalent to 249 g (68 percent yield) based on KF.

Example 6

To a 3 L flask, equipped as described in Example 1, was charged 2 L of N-methyl pyrrolidinone (NMP) which was heated under 20 mm (2,66 kPa) at 120°C to dry the system. About 200 ml of NMP and water were removed. The vacuum was released and 174 g (3 moles) of KF (dried *in vacuo* 24 hours at 140°—160°C and then pulverized), 20 g of $K_2CO_3$ (anhydrous) and 800 g (4 moles) of 3-chloro-2-fluoro-5-trifluoromethylpyridine were added slowly to the flask over a 6 hour period. Heating was resumed with vigorous stirring under a nitrogen atmosphere fed into the top of the reaction flask. The temperature was increased to 190°—195°C and the distillation head temperature was observed to drop from 138°C to 104°C over 1/2 hour, at which point a slow product takeoff (40/1 reflux to takeoff) was begun. Over the next 22 hours 532 g of distillate were recovered. The distillate contained 85 percent 2,3-difluoro-5-(trifluoromethyl)pyridine equivalent to 452 g (82 percent yield) based on KF.

Example 7

To a 3 L flask, equipped as described in Example 1, was charged 2 L of N-methyl pyrrolidinone (NMP) which was recovered from example 8 by removing the KCl salt formed in the reaction by filtration and

returning the solvent to the reaction flask. 174 Grams (3 moles) of KF (dried *in vacuo* 24 hours at 140°—160°C and then pulverized), 20 g of $K_2CO_3$ (anhydrous) and 800 g (4 moles) of 3-chloro-2-fluoro-5-(trifluoromethyl)pyridine were added slowly to the flask over an 8 hour period. Heating was resumed with vigorous stirring under a nitrogen atmosphere fed into the top of the reaction flask. The temperature was increased to 190°—195°C and the distillation head temperature was observed to drop from 138°C to 104°C over 1/2 hour, at which point a slow product takeoff (40/1 reflux to takeoff) was begun. Over the next 24 hours 596 g of distillate were recovered. The distillate contained 75 percent 2,3-difluoro-5-(trifluoromethyl)-pyridine equivalent to 447 g (81 percent yield) based on KF.

Example 8

Preparation of 2,3-Difluoro-3-(trifluoromethyl)pyridine

A 2 liter 3-necked flask was equipped with an efficient stirrer, a 1 inch ID seven tray glass Oldershaw (sieve plate) distillation column having a vapor fraction cutter condenser and a water-jacketed receiver. The apparatus was also fitted with a thermometer, temperature controller, 250 W infrared heat lamp, and an efficient reduced pressure control device. To this apparatus was charged 1200 ml of dimethylsulfoxide (DMSO). The mixture was heated with stirring under a pressure of 150 mm Hg (19,99 kPa) to reflux, and *ca.* 35 ml of the solvent was distilled at 136—139°C/19,99 kPa to dry the system. The vacuum was released, and there was added 213 g (1.4 g-mol) of cesium fluoride, which had been dried *in vacuo* at 250° for 24 hours and then pulverized, followed by 6 g of potassium carbonate and 199.5 g (1.0 g-mol) of 2-fluoro-3-chloro-5-(trifluoromethyl)pyridine. The pressure was again reduced to 150 mm Hg (19,99 kPa), and the mixture was heated with vigorous stirring to reflux. The distillation head temperature was observed to drop to 70°/19,99 kPa after 30 minutes. Distillation was then begun and maintained at 5:1 reflux over the next 4 1/2 hours, during which time 163.7 g of liquid was removed, b.p. 67—74°/19,99 kPa; during the final 15 minutes of the reaction, the head temperature increased rapidly to 136°C. The pot temperature was steadily increased from an initial 122°C to 140°C over the five hours of reaction time. Gas-liquid phase chromatograph (glpc) analysis of the product showed the presence of 144.7 g (79 percent yield) of 2,3-di-fluoro-5-(trifluoromethyl)pyridine and 9 percent recovery of 3-chloro-2-fluoro-5-(trifluoromethyl)pyridine. The net yield of the desired product was therefore 87 percent at 91 percent conversion.

Example 9

Preparation of 5-chloro-2,3-difluoropyridine from 2,3,5-trichloropyridine

To a 1 liter 3-necked flask equipped as described in Example 1 was charged 600 ml of DMSO, and 20 ml of solvent was distilled (b.p. 140—142°/19,99 kPa) to dry the system. The vacuum was released and there was added 224 g (1.47 g-mol) of CsF which had been dried *in vacuo* at 250°C for 16 hours and then pulverized, followed by 3 g of potassium carbonate and 104 g (0.57 g-mol) of 2,3,5-trichloropyridine. The mixture was heated to reflux at 200 mm pressure for 2 hours, during which time the head temperature was observed to drop from 122° to 100°C. The pressure was then reduced from 177 mm (23,59 kPa), and slow distillation over the next 7 hours while the pot temperature was being raised from 133°C to 142°C gave a total of 60.2 g of material, b.p. 96—108°C/23,32 kPa. Glpc analysis indicated the presence of 45.6 g of 5-chloro-2,3-difluoropyridine and 7.9 g of 3,5-dichloro-2-fluoropyridine. Redistillation yielded 43 g of the former as a colorless oil, b.p. 83—84°C/19,99 kPa and 7.9 g of a semisolid fraction, b.p. 122—123°C. 19,99 kPa, which was determined by glpc analysis to be 93.5 percent pure 3,5-dichloro-2-fluoropyridine. The proton and fluorine NMR spectra were consistent with the assigned structures.

Example 10

Preparation of 5-chloro-2,3-difluoropyridine from 3,5-dichloro-2-fluoropyridine

To a 1 liter 3-necked flask equipped as described in Example 1 was charged 600 ml of DMSO, and the material was heated to distill *ca.* 15 ml of solvent to dry the system (b.p. 140°C/26,66 kPa). The vacuum was released and 186 g (1.22 g/mol) CsF (dried 16 hours at 200°C *in vacuo*), 3 g of potassium carbonate and 133 g (0.80 g-mol) of 3,5-dichloro-2-fluoropyridine was added. The mixture was heated to reflux at 26,66 kPa; the head temperature dropped from an initial 126°C to 97°C/26,66 kPa over 30 minutes, and distillation was then carried out over 5.25 hours at 97—106°C/26,66 kPa, pot temperature 139—144°C, to yield 68.8 g of material. Distillation was interrupted at this point and the reaction was shut down overnight. Continuation the next day yielded an additional 21.7 g of material, b.p. 96—118°C/26,66 kPa over 3 more hours while the pot temperature was raised from 142°C to 145°C. Analysis of the product showed the presence of a total of 74.8 g of 5-chloro-2,3-difluoropyridine and 10.1 g of the starting material, for a yield of 67 percent at 92 percent conversion. Redistillation gave a recovery of 74 g of 5-chloro-2,3-difluoropyridine, b.p. 84—86°/19,99 kPa (135—136°C/101,32 kPa) which was 99.1 percent pure by glpc analysis.

Example 11

Preparation of 2,3-difluoro-5-(trifluoromethyl)pyridine showing the effect of not drying the system on the yield

The apparatus and procedure was essentially the same as in Example 10 excepting that the CsF was not pre-dried and the system-drying step (distillation of DMSO prior to adding the other reactants) was omitted. The initial head temperature was 120°C/19,99 kPa, and distillation of the product as it was formed

was at 66—82°C/19,99 kPa over 5 hours. The distillate weighed 140.2 g and was found to contain 130.7 g of 2,3-difluoro-5-(trifluoromethyl)pyridine and 2.2 g of starting material (74 percent yield at 97 percent conversion).

Example 12

To the apparatus described in Example 1 was charged 600 ml of DMSO, and *ca.* 30 ml of DMSO were distilled at 136°C/19,99 kPa to dry the system. There was then added 160 g (1.05 mole) of dry CsF, 100 g (0.46 mole) of 2,3-dichloro-5-(trifluoromethyl)pyridine and 3 g of $K_2CO_3$. The mixture was heated with stirring to reflux at 150 mm (19,99 kPa) for 1 hour, during which time the head temperature dropped to *ca.* 90°C. Slow distillation was then begun, there being recovered 20.2 g of product consisting mostly of 2,3-difluoro-5-(trifluoromethyl)pyridine over 5 hours.

Distillation was interrupted at this point and the mixture was allowed to stir at 135°C overnight. Continuation of the distillation gave an additional 63.7 g of material b.p. 58—137°/19,99 kPa. Analysis of the combined products by glpc showed the presence of 39.2 g of 2,3-difluoro-5-(trifluoromethyl)pyridine and 25.3 g of 3-chloro-2-fluoro-5-(trifluoromethyl)pyridine.

**Claims**

1. A method of preparing a beta-fluoropyridine compound from a beta-halopyridine compound which comprises contacting, under reactive conditions in a liquid medium, an alpha- or gamma-halo-substituted beta-halopyridine with an effective amount of KF or CsF, characterized in that the reaction is carried out in a polar aprotic diluent at elevated temperatures, while removing the beta-fluoropyridine product essentially as it is formed, and optionally adding the alpha- or gamma-halo-substituted beta-halopyridine as the product beta-fluoropyridine is removed.

2. Method of Claim 1 wherein the alpha- or gamma-halo-substituent is fluorine.

3. Method of Claim 1 wherein the alpha- or gamma-halo-substituent is chlorine and the product is an alpha, beta- or beta, gamma-difluoropyridine compound.

4. Method of Claim 2 wherein the beta-substituent is chlorine.

5. Method of Claim 3 wherein the beta-substituent is chlorine and the product is an alpha, beta- or beta, gamma-difluoropyridine compound.

6. Method of Claim 1 wherein the alpha-halo-substituted beta-halopyridine compound is V, the beta-fluoropyridine product is VI, or the gamma-halo-substituted beta-halopyridine compound is VII and the beta-fluoropyridine product is VIII:

V 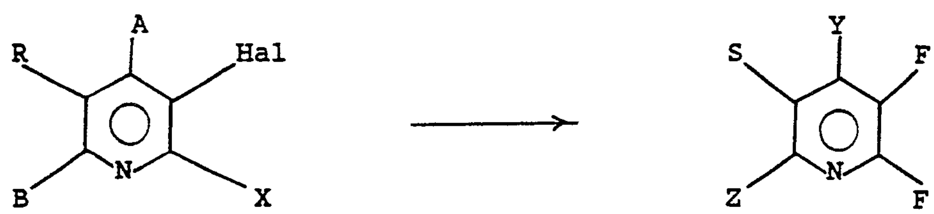 VI

VII 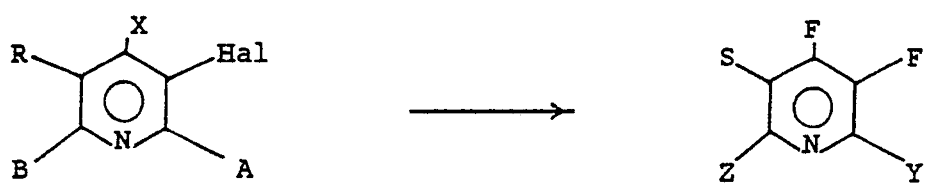 VIII

Where Hal = Cl, Br, or I; X = F or Hal; A and B are individually H, $CH_3$, $CF_3$, F or Hal; R = H, $CF_3$, $CH_3$, F, Hal or CN; Y is A or F with the proviso that Y becomes F if A is Hal; Z is B or F with the proviso that Z becomes F if B is Hal; and S is R or F with the proviso that S becomes F when R is Hal and either A or B is F or Hal.

7. Method of Claim 6 wherein formula V is 3-chloro-2-fluoro-5-(trifluoromethyl)pyridine and the product VI is 2,3-difluoro-5-(trifluoromethyl)pyridine.

8. Method of Claim 6 wherein V is 2,3-dichloro-5-(trifluoromethyl)pyridine.

9. Method of Claim 6 wherein the fluoride salt is KF and the reaction is carried out in the presence of (a) a phase-transfer catalyst and optionally (b) an acid scavenger.

10. Method of Claim 6 wherein the polar aprotic diluent is selected from the group comprising dimethylsulfoxide (DMSO), dimethylformamide (DMF), dimethylacetamide, diethylacetamide, methyl isobutyl ketone, hexamethylphosphoric acid triamide, tetramethylurea, sulfolane (tetramethylenesulfone), and N-methylpyrrolidinone (NMP).

11. Method of Claim 9 wherein the phase-transfer catalyst is a quaternary ammonium salt, a crown ether, or tris(3,6-dioxaheptyl)amine.

**Patentansprüche**

1. Verfahren zur Herstellung einer beta-Fluorpyridinverbindung aus einer beta-Halopyridinverbindung, umfassend Kontaktieren eines alpha- oder gamma-halosubstituierten beta-Halopyridins mit einer wirksamen Menge von KF oder CsF unter reaktiven Bedingungen in einem flüssigen Medium, dadurch gekennzeichnet, daß die Reaktion in einem polaren aprotischen Verdünnungsmittel bei erhöhten Temperaturen ausgeführt wird, während das beta-Fluorpyridinprodukt im wesentlichen sobald es gebildet wird, entfernt wird und gegebenenfalls das alpha- oder gamma-halosubstituierte beta-Halopyridin zugegeben wird, während das Produkt beta-Fluorpyridin entfernt wird.

2. Verfahren nach Anspruch 1, worin der alpha- oder gamma-Halosubstituent Fluor ist.

3. Verfahren nach Anspruch 1, worin der alpha- oder gamma-Halosubstituent Chlor ist und das Produkt eine alpha, beta- oder beta, gamma-Difluorpyridinverbindung ist.

4. Verfahren nach Anspruch 2, worin der beta-Substituent Chlor ist.

5. Verfahren nach Anspruch 3, worin der beta-Substituent Chlor und das Produkt eine alpha, beta- oder beta, gamma-Difluorpyridinverbindung ist.

6. Verfahren nach Anspruch 1, worin die alpha-halo-substituierte beta-Halopyridinverbindung V ist, das beta-Fluorpyridinprodukt VI, oder die gamma-halo-substituierte beta-Halopyridinverbindung VII und das beta-Fluorpyridinprodukt VIII ist:

V

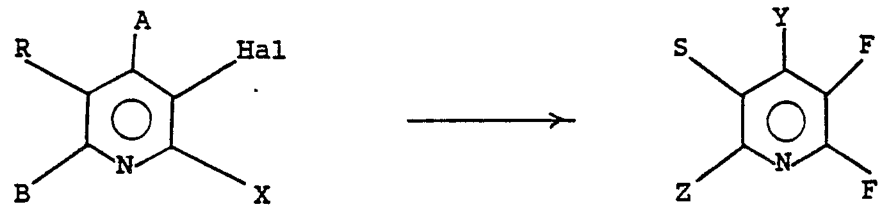

VI

VII

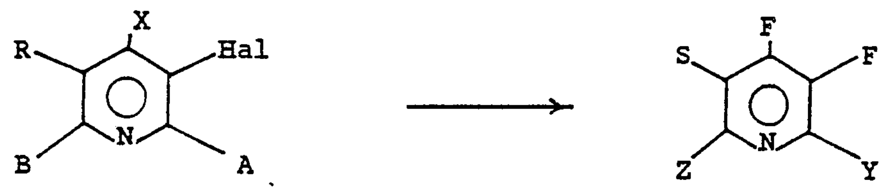

VIII

worin Hal = Cl, Br oder I; X = F oder Hal; A und B unabhängig voneinander H, $CH_3$, $CF_3$, F oder Hal sind; R = H, $CF_3$, $CH_3$, F, Hal oder CN; Y A oder F ist mit der Maßgabe, daß Y F wird, wenn A Halogen ist; Z B oder F ist, mit der Maßgabe, daß Z F wird, wenn B Hal ist; und S R oder F ist mit der Maßgabe, daß S F wird, wenn R Hal ist und entweder A oder B F oder Hal sind.

7. Verfahren nach Anspruch 6, worin Formel V 3-Chlor-2-fluor-5-(trifluormethyl)pyridin und das Produkt VI 2,3-Difluor-5-(trifluormethyl)pyridin ist.

8. Verfahren nach Anspruch 6, worin V 2,3-Dichlor-5-(trifluormethyl)pyridin ist.

9. Verfahren nach Anspruch 6, worin das Fluoridsalz KF ist und die Reaktion in Anwesenheit (a) eines Phasentransferkatalysators und gegebenenfalls (b) eines Säurefängers ausgeführt wird.

10. Verfahren nach Anspruch 6, worin das polare aprotische Verdünnungsmittel ausgewählt ist aus der Gruppe, umfassend Dimethylsulfoxid (DMSO), Dimethylformamid (DMF), Dimethylacetamid, Diethylacetamid, Methylisobutylketon, Hexamethylphosphorsäuretriamid, Tetramethylharnstoff, Sulfolan (Tetramethylensulfon) und N-methylpyrrolidon (NMP).

11. Verfahren nach Anspruch 9, worin der Phasentransferkatalysator ein quaternäres Ammoniumsalz, ein Kronenether oder Tris(3,6-dioxaheptyl)amin ist.

**Revendications**

1. Procédé de préparation d'une béta-fluoropyridine, à partir d'un composé béta-halogénopyridine, comprenant la mise en contact, dans des conditions de réaction, dans un milieu liquide, d'une béta-halogénopyridine substituée en alpha ou gamma par un halogène, avec une quantité efficace de KF ou de CsF, caractérisé en ce que la réaction est effectuée dans un diluant aprotique polaire à des températures élevées, le produit béta-fluoropyridine étant éliminé au fur et à mesure de sa formation, et la béta-halogénopyridine substituée en alpha ou gamma par un halogéne étant éventuellement ajoutée pendant que la béta-fluoropyridine est éliminée.

2. Procédé selon la revendication 1, dans lequel le substituant halogène en alpha ou gamma est le fluor.

3. Procédé selon la revendication 1, dans lequel le substituant halogène en alpha ou gamma est le chlore et le produit est un composé alpha,béta- ou béta,gamma-difluoropyridine.

4. Procédé selon la revendication 2, dans lequel le substituant en béta est le chlore.

5. Procédé selon la revendication 3, dans lequel le substituant en béta est le chlore et le produit est un

composé alpha,béta- ou béta,gamma-difluoropyridine.

6. Procédé selon la revendication 1, dans lequel le composé béta-halogénopyridine substitué en alpha par un halogéne est V, le produit béta-fluoropyridine est VI, ou le composé béta-halogénopyridine substitué en gamma par un halogéne est VII et le produit béta-fluoropyridine est VIII:

V 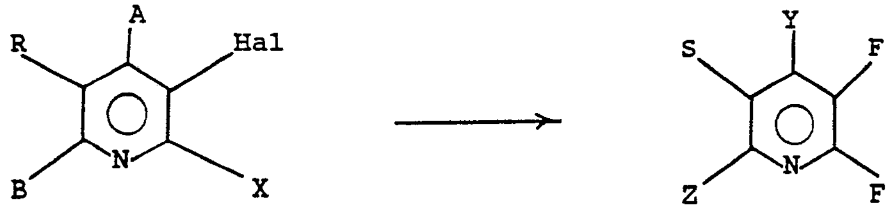 VI

VII 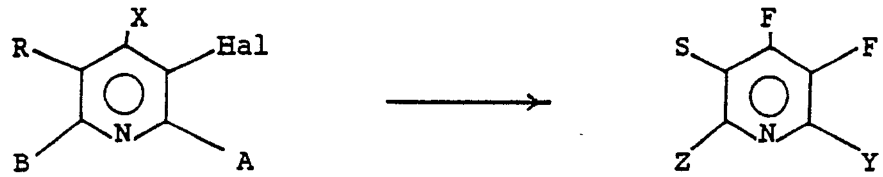 VIII

où Hal = Cl, Br ou I; X = F ou Hal; A et B représentent individuellement H, $CH_3$, $CF_3$, F ou Hal; R = H, $CF_3$, $CH_3$, F, Hal ou CN; Y est A ou F, étant entendu que Y devient F si A est Hal; Z représente B ou F, étant entendu que Z devient F si B est Hal; et S représente R ou F, étant entendu que S devient F lorsque R est Hal et que A ou B représente F ou Hal.

7. Procédé selon la revendication 6, dans lequel la formule V représente la 3-chloro-2-fluoro-5-(trifluorométhyl)pyridine et le produit VI est la 2,3-difluoro-5-(trifluorométhyl)pyridine.

8. Procédé selon la revendication 6, dans lequel la formule V représente la 2,3-dichloro-5-(trifluorométhyl)pyridine.

9. Procédé selon la revendication 6, dans lequel le sel fluorure est KF et la réaction est effectuée en présence (a) d'un catalyseur de transfert de phase et éventuellement (b) d'un agent de fixation des acides.

10. Procédé selon la revendication 6, dans lequel le diluant aprotique polaire est choisi dans le groupe comprenant le diméthylsulfoxyde (DMSO), le diméthylformamide (DMF), le diméthylacétamide, le diéthylacétamide, la méthylisobutylcétone, l'héxaméthylphosphorotriamide, la tétraméthylurée, le sulfolane(tétraméthylènesulfone), et la N-méthylpyrrolidone (NMP).

11. Procédé selon la revendication 9, dans lequel le catalyseur de transfert de phase est un sel d'ammonium quaternaire, un éther-couronne, ou la tris(3,6-dioxaheptyl)amine.